# EUROPEAN PATENT APPLICATION

(11) **EP 1 790 381 A1**
(43) Date of publication of application: **30.05.2007**
(21) Application number: 06122965.4
(22) Date of filing: 25.10.2006
(51) Int. Cl.: A61N 1/36, A61H 19/00

(54) **Electric stimulation sexual enhancement appliance**

(30) Priority: 21.11.2005 US 282743
(71) Applicant: Yang, Jin-Shoei, 514 Changhua Conty (TW)
(72) Inventor: Yang, Jin-Shoei, 514 Changhua Conty (TW)
(74) Representative: Jannig, Peter

(57) **Abstract**

A percutaneous electric stimulation sexual enhancement appliance includes a built-in control mechanism containing one central processing unit (CPU), an oscillation unit; a display unit; a control unit; an output unit; and a reset unit to deliver a medium to low frequency with a power lower than the general power to stimulate (be applied on) a specific (sensitive) part of human body.

## Description

### FIELD OF THE INVENTION

The present invention relates to a chip packaging structure, and more particularly to a medium to low frequency electric stimulation sexual enhancement appliance, and more particularly, to one that outputs medium to low frequency lower than the general power to stimulate (be applied on) specific (sensitive) part of a human body.

### BACKGROUND OF THE INVENTION

As taught in an application published under #580 383 of ROC (Taiwan) Gazette, a "Low Frequency Electric Stimulation Appliance for Breast Massage", the prior art is essentially comprised of an electric stimulation plate provided on a conductive film, copper foil plate, or conductive rubber material that contains one or a plurality of pair of positive and negative polarities connected in series or parallel with those polarities located at where in corresponding to those critical points on human body believed to be lethally vulnerable or where nerve centers are located.

In practice, the prior art is attached to the critical point on one's breast to deliver low frequency electric stimulation; however, the prior art relates to a conventional low frequency electric stimulation circuit that generates comparatively higher low frequency electric stimulation power, the user may feel discomfort when the prior art is applied to his/her breast or other specific (sensitive) part. The prior art of the conventional low frequency electric stimulation circuit fails to meet the needs of its user.

### SUMMARY OF THE INVENTION

A primary object of the present invention is to provide an appliance that outputs medium to low frequency lower than the general power to stimulate (be applied on) a specific (sensitive) part of the human body.

To achieve the purpose, the present invention related to a medium to low frequency electric stimulation appliance is comprised of a built-in control mechanism containing one central processing unit (CPU) connected to a source to control and execute operation by delivering medium to low frequency electric stimulation function; an oscillation unit connected to the CPU to drive the CPU to execute the operation as required; a display unit connected to the CPU to display the operation status of the appliance; a control unit connected to the CPU to set up the output of the medium to low frequency electric stimulation as required; an output unit connected to the CPU and made of molded conductive rubber or other conductive material to receive the settings from the control unit and in turn to output medium to low frequency to the molded conductive rubber or other conductive material; and a reset unit connected to the CPU for circuit protection.

### BRIEF DESCRIPTION OF THE DRAWINGS

The structure and the technical means adopted by the present invention to achieve the above and other obj ects can be best understood by referring to the following detailed description of the preferred embodiments and the accompanying drawings, wherein
Fig. 1 is a schematic view showing a framework of the present invention.
Fig. 2 is a schematic view showing the process flow of the status of use of the present invention.
Fig. 3 is a schematic view showing a circuit of the present invention.
Fig. 4 is a schematic view showing another circuit of the present invention.
Fig. 5 is schematic view of a first preferred embodiment of the present invention.
Fig. 6 is schematic view of a second preferred embodiment of the present invention.
Fig. 7 is schematic view of a third preferred embodiment of the present invention.

### DETAILED DESCRIPTION OF THE PREFERRED EMBODIMENTS

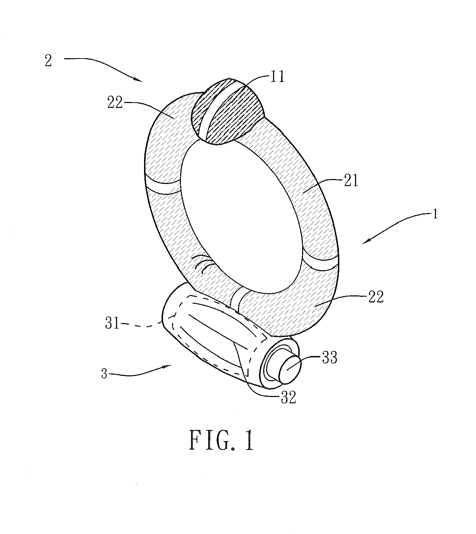

Referring to Fig. 1 for a framework of the present invention, the present invention related to a medium to low frequency electric stimulation sexual enhancement appliance is essentially comprised of a main unit and a control mechanism 7. The control mechanism 7 includes a central processing unit (CPU) 1, an oscillation unit 2, a display unit 3, a control unit 4, an output unit 5, and a reset unit 6 to output medium to low frequency lower than the general power to achieve the purpose of stimulating (being applied on) a specific (sensitive) part of human body.

The CPU 1 in the control mechanism 7 is connected to a source 11 to control and execute the operation by delivering electric stimulation at medium to low frequency; and the CPU 1 permits writing of applications for the operation control and delivery of electric stimulation at medium to low frequency as applicable.

The oscillation unit 2 is connected to the CPU 1 to drive the CPU 1 to execute the operation as desired.

The display unit 3 is connected to the CPU 1 to display the status of the appliance and may be made in the form of a screen, audible device or LED or the combination of the screen, audible device and LED.

The control unit 4 is connected to the CPU 1 to set up the input of a medium to low frequency electric stimulation required for the subsequent output; and the control unit 4 is connected to a keypad 41.

The output unit 5 is connected to the CPU 1 and further connected to a molded conductive rubber or conductive material 51 to receive the settings from the control unit 4 and to output the medium to low frequency as required to the molded conductive rubber or conductive material 51. The output unit 5 is provided with a positive and a negative output ends to define the positive and the negative polarities for the molded conductive rubber or the conductive material 51.

The reset unit 6 is connected to the CPU 1 to protect the circuit. During the downtime of the appliance, the reset unit 6 serves for restoration of circuit.

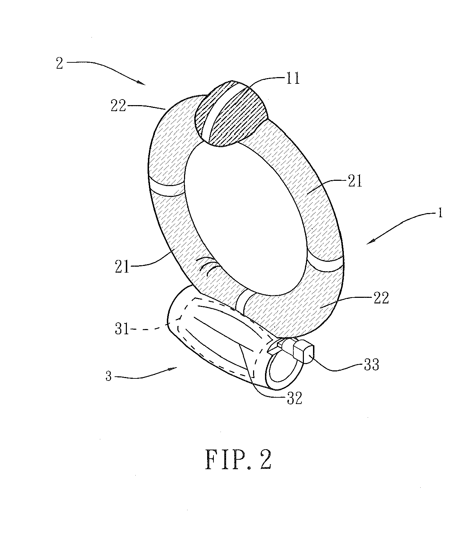

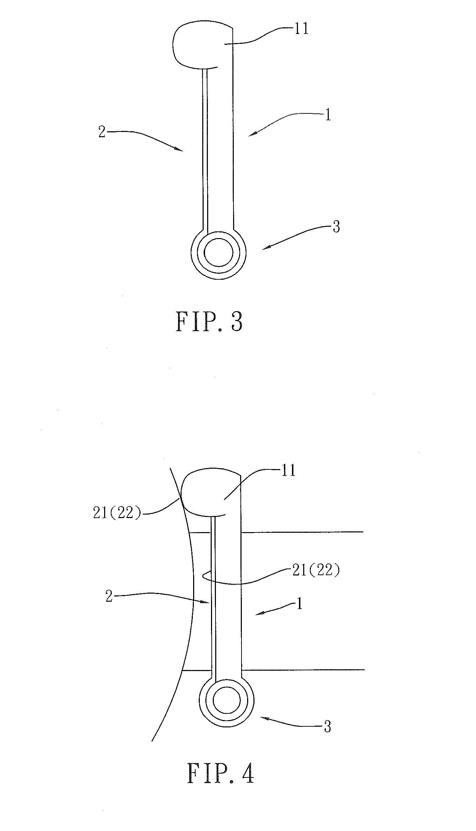

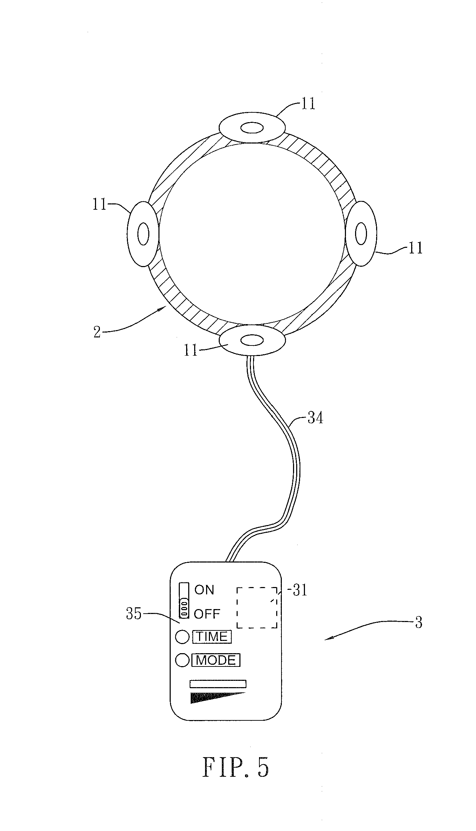

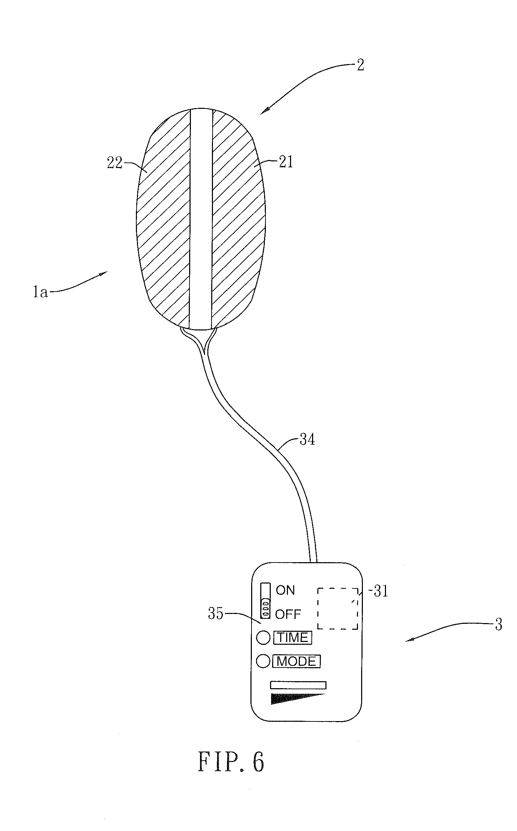

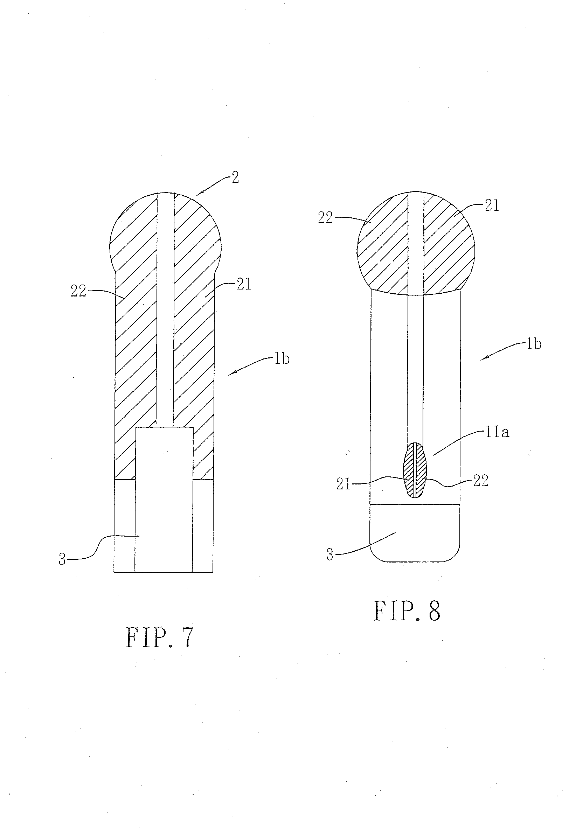

Now referring to Figs. 2 through 7 , the control mechanism 7 of the present invention is adapted to the main unit as applicable, and the main unit is made in the form of a rod vibrator 8 as illustrated in Fig. 5, a ring vibrator 8a as illustrated in Fig. 6 or an oval vibrator 8b as illustrated in Fig. 7 while allowing the molded conductive rubber or the conductive material 51 wrapping around the main unit 8, 8a or 8b. The main unit 8, 8a or 8b is provided with a circuit as illustrated in Fig. 3 or Fig. 4. In use, the keypad 41 of the control unit 4 is used to input the medium to low frequency as desired for subsequent output of electric stimulation so to permit changes and combinations of various frequencies to generate continuous, intermittent or alterative electric stimulation. The module so set up for output is displayed on the display unit 3. Upon completing the setup, the appliance of the present invention is ready for use as desired. A loop is created once the skin of the user contacts at the molded conductive rubber or conductive material 51 of the output unit 5 and the signals of the loop are transmitted to the CPU 1 for it to release the medium to low frequency electric stimulation depending on the model set up by the control unit 4 and through the drive by the oscillation unit 2 to the molded conductive rubber of the conductive material 51 of the output unit 5. The skin of the user is electrically stimulated to generate comfortable sense of touch when conducted by the positive and the negative polarities defined by the molded conductive rubber of the conductive material 51.

The prevent invention providing a sexual appliance delivering electric stimulation at medium to low frequency lower than the general power to stimulate (be applied on) a specific (sensitive) part of the human body complies with the requirements of more advanced, practical and meeting user needs, and the application for a patent is duly filed accordingly. However, it is to be noted that the preferred embodiments disclosed in the specification and the accompanying drawings are not limiting the present invention; and that any construction, installation, or characteristics that is same or similar to that of the present invention should fall within the scope of the purposes and claims of the present invention.

## Claims

1. A percutaneous electric stimulation sexual enhancement appliance includes
a main unit;
a control mechanism built in the main unit including a central processing unit (CPU), connected to a source to control the operation of the appliance and to execute release of medium to low frequency for electric stimulation;
an oscillation unit connected to the CPU to drive the CPU to execute the operation as desired;
a display unit connected to the CPU to display the status of the appliance;
a control unit connected to the CPU to set up the input of medium to low frequency for subsequent output to provide electric stimulation;
an output unit connected to the CPU, and further connected with molded conductive rubber or other conductive materials to receive the settings from the control unit and to output the medium to low frequencies to the molded conductive rubber or other conductive material that in turn wrapping around the main unit; and
a reset unit connected to the CPU for circuit protection.

2. A percutaneous electric stimulation sexual enhancement appliance of Claim 1, wherein the appliance is related to a ring object.

3. A percutaneous electric stimulation sexual enhancement appliance of Claim 1, wherein the appliance is related to a rod object.

4. A percutaneous electric stimulation sexual enhancement appliance of Claim 1, wherein the appliance related to an oval object.

5. A percutaneous electric stimulation sexual enhancement appliance of Claim 1, wherein the CPU permits the writing of applications depending on the operation control and the delivery of the medium to low frequency electric stimulation as applicable.

6. A percutaneous electric stimulation sexual enhancement appliance of Claim 1, wherein the control unit is connected with a keypad.

7. A percutaneous electric stimulation sexual enhancement appliance of Claim 1, wherein the output unit is provided with a pair of positive and negative outputs to define the positive and the negative polarities for the molded conductive rubber or conductive material with where between the positive and the negative polarities indicating insulated status.
